# EUROPÄISCHE PATENTSCHRIFT

(11) **EP 0 724 452 B1**
(45) Veröffentlichungstag und Bekanntmachung des Hinweises auf die Patenterteilung: **24.05.2000**
(21) Anmeldenummer: 94928852.6
(22) Anmeldetag: 30.09.1994
(51) Int. Cl.: A61K 38/13, A61K 9/48, A61K 47/22

(54) **PHARMAZEUTISCHE ZUSAMMENSETZUNG MIT CYCLOSPORIN A, EINEN VITAMIN E DERIVAT UND EINEN EMULGATOR**
PHARMACEUTICAL COMPOSITION CONTAINING CYCLOSPORINE A, A VITAMINE E DERIVATIVE AND AN EMULSIFIER
COMPOSITION PHARMACEUTIQUE CONTENANT DE LA CYCLOSPORINE A, UN DERIVE DE LA VITAMIE E ET UN EMULSIFIANT

(30) Priorität: 22.10.1993 DE 4336163
(43) Veröffentlichungstag der Anmeldung: 07.08.1996
(73) Patentinhaber: HEXAL AG, D-83607 Holzkirchen (DE)
(72) Erfinder: KLOKKERS, Karin, D-83607 Holzkirchen (DE); FISCHER, Wilfried, D-83607 Holzkirchen (DE)
(74) Vertreter: Boeters, Hans Dietrich, Dr.
(86) Internationale Anmeldenummer: EP9403274
(87) Internationale Veröffentlichungsnummer: WO9511039

(56) Entgegenhaltungen:
- WO-A-87/02219
- LANCET (UNITED KINGDOM), 1991, VOL. 338, NO. 8761, PAGE(S) 212-215, Sokol R.J. et al 'Improvement of cyclosporin absorption in children after liver transplantation by means of water-soluble vitamin E'
- INN. MED. (GERMANY), 1992, VOL. 19, NO. 2, PAGE(S) 52-55, Gutzler F. 'BIOVERFUGBARKEIT VON CICLOSPORIN'

## Beschreibung

### 1. Gebiet der Erfindung

Die Erfindung bezieht sich auf pharmazeutische Zusammensetzungen, die eine wirksame Menge Cyclosporin A in Kombination mit einem Vitamin E-Derivate enthält.

### 2. Stand der Technik

Vitamin E beeinflußt den Arachidonsäurestoffwechsel im Sinne einer Hemmung der Prostaglandin-, Thromboxan- und Leukotrienbiosynthese und einer Erhöhung der Prostacyclinbildung. Diese Eigenschaften stehen mit einer biologischen Entzündungshemmung und mit thrombotischen Erkrankungen in Zusammenhang (Machlin, Vi-ta-min E.; in: Machlin, Handbook of Vitamins: Nutritional, Biochemical and Clinical Aspects, Seiten 99-145, Marcel Dekker, New York, 1984). Vitamin E kann nach oraler Einnahme ebenfalls die Aktivitt nicht steroidaler entzündungshemmender Arzneistoffe fördern (Bertolini et al., Rivista di Pharmakologia et Therapia 8, Seiten 27-34 (1982); Klein & Blankenhorn, Vergleich der klinischen Wirksamkeit von Vitamin E und Diclofenac Natrium bei Spondylitis Ancylosans (Morbus Bechterew), Vitaminspur 2, Seiten 137-142 (1987)). Nach topischer Anwendung permeiert Vitamin E sehr gut das Stratum Corneum. Es wurden quantitative Resorptionsstudien an der Haut von Versuchstieren durchgeführt. So wurde 16 Stunden nach Applikation von 300 µg einer 5-proz. Vitamin E-Lösung in Ethanol pro cm² 10,7 % Vitamin E in der Hornschicht und ca. 40,9 % in darunterliegenden Hautschichten wiedergefunden (Djerassi et al., Vitamin E: Biochemical function and its role in cosmetics, Drug & Cosmetic Industry 13: Nr. 1, Seiten 29-31, 34, 78 (1986)). Lokal appliziert wirkt Vitamin E als membranstabilisierendes Antioxydans und hemmt die Freisetzung von Histamin und hydrolytischen Enzymen z. B. aus den Mastzellen und den Lysosomen durch Stabilisierung ihrer Membranen. Ebenfalls hemmt es die Synthese bestimmter Prostaglandine, desaktiviert Sauerstoffnadikale und entgiftet entsprechende Folgeprodukte (Sies, Bildung von Superoxidradikalen und Peroxiden; in: Superoxiddismutase - Biochemie und therapeutscher Einsatz; Herausgeber Puhl & Ries, Perimed Verlag, Erlangen, 1982). Zudem erhöht Vitamin E die Hautfeuchigkeit und wirkt quasi als Okklusionsmittel.

Cyclosporin A ist ein zyklisches, wasserunlösliches, unpolares Undekapeptid. Die Verbindung ist ein gut wirksames Immunsuppressivum, gewonnen aus Pilzkulturen (Cane et al., Transplant TROC. 13: 349-358 (1981); Ferguson et al., Surgery 92: 175-182 (1982)). Der Arzneistoff wird zur Vorbeugung der Abstoßung transplantierter allogener Organe eingesetzt (Bennett & Norman, Arzn. Rev. Med. 37, 215-224 (1986); Van Basen, Surg. Clin. North Am. 66, 435-449 (1986)). Seine immunsuppressive Wirkung beruht auf einer selektiven Hemmung der Zellfunktion, die ein Überleben von z. B. Herztransplantaten ohne Myelozytensuppression erlaubt (Myers et al, New England Journal of Medicine 311: 699 (1984)). Zusätzlich zur Verwendung bei Transplantationen haben neuere klinische Prüfungen gezeigt, daß Cyclosporin A bei der Behandlung einer großen Anzahl von Autoimmunerkrankungen wirksam ist. Beispielsweise wurden klinische Prüfungen zur Behandlung von Polymyositis, systemischem Lupus Erythematodes, Pheumatischer Arthritis oder sogar von jugendlichem insulinabhängigen Diabetes durchgeführt (siehe die entsprechenden Kapitel in: Cyclosporine in Autoimmune Diseases, Herausgeber Schindler, Springer Verlag, Berlin 1985).

Cyclosporin ist ein lipophiles Molekül mit einem Molekulargewicht von 1202 Dalton. Aufgrund der schlechten Wasserlöslichkeit und der hohen Lipophilie des Cyclosporin A besitzen dessen pharmazeutische Zusammensetzungen mit üblichen festen oder flüssigen pharmazeutischen Trägerstoffen oft Nachteile. So werden die Cyclosporine aus solchen Zusammensetzungen nicht zufriedenstellend resorbiert (Cavanak & Sucker, Formulation of Dosage Forms, Prog. Allergy 38, 65-72 (1986)), oder die Zusammensetzungen werden nicht gut vertragen, oder sie sind bei der Lagerung nicht genügend stabil, beispielsweise gegen die Auskristallisation des Cyclosporins.

So ist beispielsweise aus The Lancet, 338/1991/212-215 bekannt, eine wässrige Lösung von Tocopherolpolyethylenglykol-1000-Succinat (LIQUI-E) mit Cyclosporin A zu kombinieren, wobei man aus mitveröffentlichten Daten schließen kann, daß eine Wirkstoff-Suspension appliziert wurde.

Ferner ist oft die gelöste Konzentration in Relation zur Dosis von bis zu 1 g täglich niedrig, z. B. nur 3 %, was die Einnahme von 30 g Lösung bedeutet. Eine höhere Löslichkeit wird in DE-B-2 907 460 angeführt, worin eine Lösung von Cyclosporin in Pflanzenöl wie Olivenöl oder Maisöl, Ethanol und einem Emulgator aus einem nichtionischen Ester eines Triglyzerids mit einem Polyalkylenglycol beschrieben wird. Beispiele der von diesem Patent bevorzugt angegebenen Zusammensetzungen sind Trinklösung, Trinkemulsion, Injektionslösung und in Kapseln befindliche Lösung.

Die Verabreichung der obigen Zusammensetzung erfolgt vorzugsweise intramuskulär, oder subkutan, oder insbesondere oral. Cyclosporin A, appliziert mit obigen Arzneiformen, zeichnet sich durch eine gute Bioverfügbarkeit aus. Nach der Resorption bindet die Substanz schnell an Plasmaproteine und hat eine terminale Halbwertszeit von 24 Stunden. Es wird zu einem hohen Prozentsatz in der Leber metabolisiert, wobei die biliäre Exkretion die Haupteliminationsroute ist (Beverige, Cyclosporin A; in: Proceedings of International Symposium, Cambridge, Herausgeber White, Seiten 35-44 (1982)).

Trotz des großen Wertes als Immunsuppressivum ist die klinische Verwendung von Cyclosporin durch die Hauptnebenwirkung bei der chronischen Anwendung limitiert, die in der Nephrotoxizität des Wirkstoffes selber besteht (Van Buren, Surg. Clin. North Am. 66, 435-449 (1986)). Auch in etwa 80 % der Nierentransplantationspatienten tritt Nierentoxizität auf (Kahan, Dial. Transplant. 12: 620-30 (1983)), und zwar durch diese substanzimmanente Nebenwirkung, die zum Schutz des Tranaplantates vor der Abstoßung angewendet wird.

Häufige Nebeneffekte von Cyclosporinbehandlungen bei verschiedenen Autoimmunerkrankungen schließen der Nephrotoxizität, die Hypertension, Hyperkaliämie, Hyperurikoämie, Hepatotoxizität, Anämie, Hypertrichiose, Gingivalhyperplasie, gastrointestinale Nebenwirkungen, Tremor und Paresthesien ein (Von Graffenried et al., Cyclosporine in Autoimmune Diseases, Herausgeber Schindler, Springer Verlag, Berlin, Seiten 59-73 (1985)). Von den hier zitierten Nebenwirkungen ist die häufigste die Nephrotoxizität. Die akute, durch Cyclosporin induzierte Nephrotoxizität ist dosisabhängig und korreliert mit den Cyclosporinblutspiegeln. Sie ist reversibel nach Dosisreduktion oder nach Beendigung der Cyclosporintherapie (Chapman et al., Lancet I: 128 (1985)).

Akute Cyclosporinnephrotoxizität geht morphologisch einher mit tubulären Läsionen, die durch Einschlußkörperchen, isometrische Vakuolisierung und Mikrocalzifizierung gekennzeichnet sind (Mihatsch et al., Transplant. Proc. 15: 2821 (1983)). Dieses führt zu einer Abnahme der glomerulären Filtrationsrate, wie anhand des schnellen Anstiegs von Serumkreatinin in cyclosporinbehandelten Patienten erkannt werden kann. Ein Grund dafür könnte die Störung der Mikrozirkulation durch Interaktion von Cyclosporin mit der lokalen Prostacyclinsynthese sein (Neild et al.; in: Cyclosporine, Herausgeber Kahan, Gruen & Stratton, Orlando, Florida, Seite 182 (1984)).

Obwohl der Mechanismus der renalen Dysfunktion noch nicht vollständig aufgeklärt ist, konnte gezeigt werden, daß die renale Synthese von Thromboxan während des Fortschreitens von immun- und nicht immunvermittelten Modellen renaler Schädigung auftritt (Lianos et al., J. Clin. Invest. 72: 1439-1448 (1983); Okegawa et al., J. Clin. Invest. 71: 81-90 (1983)). Thromboxan ist ein Prostanoid und damit Metabolit der Arachidonsäure aus dem Cyclooxigenasezyklus. Die anderen Prostanoide sind Prostaglandine und Prostacycline. Prostanoide sind sehr wirksame Mediatoren, die während immunologisch erzeugter Entzündungaprozesse entstehen. Sie können grundlegend die renale Hämodynamik ändern (Morley; in: Lymphokines, Herausgeber Pic, Academic Press, New York, 4: 377-391 (1981)).

EP-A-20 305 400 beschreibt die Zusammenhänge zwischen gestörter Prostanoidsynthese und Nephrotoxizität. Danach geht die Verabreichung von Cyclosporin mit einer erhöhten Synthese von Thromboxan B2, einem Mediator von Entzündungen, einher. Cyclosporin soll dementsprechend ebenfalls die Bildung von Prostaglandinen der E-Serie, ebenfalls Entzündungsmediatoren, fördern. Die Abstoßung menschlicher Nierentransplantate konnte in Verbindung gebracht werden mit einem schnellen Anstieg von renal eliminiertem Thromboxan B2.

EP-A-0 305 400 beschreibt ferner den Einsatz von w3-ungesättigten Fettsäuren in Kombination mit Cyclosporin A zur Hemmung der Prostaglandin- bzw. Thromboxanbildung.

Ein Nachteil einer längerfristigen w3-Fettsäuregabe liegt in der Ausbildung eines Vitamin E-Mangelzustandes. Mangelzustände sind z. B. Hämolyse und eine verkürzte Lebensdauer der Erythrozyten. Im Tierexperiment führt Vitamin-E-Mangel zu degenerativen Muskelveränderungen, Kreatinurie, erhöhter Hämolyse der Erythrozyten und zur Beeinflussung bestimmter Hormone und Enzyme sowie des Protein- und Arachidonsäurestoffwechsels (Machlin, Vitamin E.; in: Machlin, Handbook of Vitamins: Nutritional, Biochemical and Clinical Aspects, Seiten 99-145, Marcel Dekker, New York, 1984).

Ein weiterer Nachteil dieser Zusammensetzung mit w3-ungesättigten Fettsäuren (Fischölen) ist die offenbar geringe zu erreichende Wirkstoffkonzentration in diesem Öl. So beschreibt EP-A-0 305 400 lediglich eine Konzentration mit 12,5 mg Cyclosporin A pro Gramm Fischöl. Bei einer üblichen täglichen Dosis von mehr als 300 mg Cyclosporin A bedeutet das eine Gesamteinnahmemenge von etwa 24 Gramm der Zubereitung, bei 1 g Cyclosporin A von 80 g Zubereitung. Dieses ist eine für Patienten unzumutbar hohe Ölmenge, die beispielsweise in Weichgelatinekapseln verkapselt zu einer täglichen Einnahme von 24 Kapseln mit 300 mg Cyclosporin A führen würde. Die parenterale Applikation per Infusion würde bei einer, optimistisch gerechnet, 10-proz. ölhaltigen Infusionsemulsion eine Menge von ca. 240 ml Emulsion mit 300 mg Cyclosporin A bedeuten, ein Volumen, das nur über längere Zeit infundiert werden kann. Beide Aspekte stehen einer chronischen Anwendung, wie sie bei Transplantationspatienten notwendig ist, absolut entgegen.

Die Formulierungen entsprechend der DE-B-2 907 460 zeichnen sich zwar durch ein sehr hohes Lösungsvermögen für Cyclosporin A aus, haben jedoch den Nachteil, daß sie nur Pflanzenöle umfassen, die keinerlei Prostaglandin- oder Thromboxansynthese-hemmende Stoffe enthalten. Das heißt, daß durch diese Zubereitungen die Nephrotoxizität des Cyclosporin A nicht gehemmt wird. Zu Innere Medizin, 19(1992)52-55 wird die Verwendung einer im Handel befindlichen parenteralen Lösung des Cyclosporin A (Sandimmun^{R}, Firma Sandoz) beschineben. Diese Lösung enthält in 1 ml 50 mg Cyclosporin A, 32,9 % Ethanol und 650 mg Cremeopher EL, ein ethoxyliertes, hydriertes Rizinusöl. Neben der Ethanolmenge von 2 g pro Anwendung, die eine Belastung für die Leber darstellt, ist nach Literaturberichten Cremeopher EL nephrotoxisch ähnlich wie Cyclosporin A selber (Thiel et al., Clin. Nephrol. 25 (Suppl. 1), 540-542 (1986); Finn et al., Renal Failure 11, 3-15 (1989)). So führt Cremeopher EL in der isolierten, perfundierten Rattenniere zu einer deutlichen renalen Vasokonstriktion mit reduziertem renalen Blutfluß und tubulärer Dysfunktion (Besarab et al., Transplantation 44, 195-201 (1987); Luke et al., Transplantation 43, 795-799 (1987)). Im weiteren verursacht Cremeopher EL anaphylaktische Reaktionen bis zum Schock (Chapuis et al., Engl. J. Med. 312, 1259 (1985), Leunissen et al., Lancet 1, 637 (1986); Magalini et al., Transplantation 42, 443-444 (1986)). Als Ursache der anaphylaktoiden Reaktion wurde Cremopher EL angesehen, da es zu Histaminliberation führt (Ennis et al., Agents Action 12, 64-80 (1982)). In einigen Therapiefällen mit der i. v. Lösung wurde die allergische Reaktion bei der ersten Anwendung am Menschen beobachtet (Friedman et al., Am J. Med 78, 343-345 (1985); Magalini et al., Transplantation 42, 443-444 (1986)). Nachteil der handelsüblichen parenteralen Zubereitung ist demnach der Inhaltsstoff Cremopher EL. Eine Formulierung ohne diesen Hilfsstoff ist anzustreben, um die obigen Nebenwirkungen zu vermeiden und die Arzneimittelsicherheit zu erhöhen.

Die günstigen immunsuppressiven Eigenschaften von Cyclosporin A werden bei der Behandlung der Psoriasis ausgenutzt. Aufgrund seines hohen Molekulargewichts und seiner sehr hohen Lipophilie ist jedoch Cyclosporin A nicht imstande, intakte Haut, speziell das Stratum Corneum, zu durchdringen. Aus diesem Grunde werden schwere Psoriasisfälle mit der oralen und parenteralen Cyclosporingabe behandelt. Nachteil dieser Anwendung sind die systemischen Nebenwirkungen auf den Kreislauf (Hypertonie) und die Nierenfunktion. Topische Zubereitungen zur Behandlung der Psorasis, womit die systemischen Nebenwirkungen reduziert würden, benötigen Resorptionsförderer wie z. B. Propylenglycol und Azone (Duncan et al., British Journal of Dermatology 123: 631-640 (1990)). Nun ist aber gerade von Azone bekannt, daß seine permeationsfördernden Eigenschaften auf eine Störung oder sogar Zerstörung der Schutzfunktion des Stratum Corneum zurückzuführen sind. Propylenglycol führt zu einem Austrocknen der Haut. Beide Substanzen würden also eher der Abheilung der Psoriasis hinderlich als förderlich sein. Aus diesem Grunde wäre eine topische Zubereitung mit einem therapeutisch ausreichenden Cyclosporin-A-Gehalt in Kombination mit den Heilungsprozeß fördernden Stoffen wünschenswert. Darüber hinaus sollte die Kombination die Permeation des Cyclosporin A durch intakte Haut fördern.

### 3. Aufgabe der Erfindung

Aufgabe der vorliegenden Erfindung ist es nun, ein vorteilhaftes Lösungsmittelsystem zu finden, das Cyclosporin A in ausreichender Menge löst, so daß es in der therapeutisch gebräuchlichen täglichen Dosierung oral eingenommen werden kann, die nephrotoxische Wirkung reduzieren kann, bei topischer Applikation sowohl die Hautpermeation fördern als auch den Heilungsprozeß bei der Behandlung der Psoriasis unterstützen kann und zudem eine parenterale Applikation mit guter Verträglichkeit gewährleistet.

### 4. Beschreibung der Erfindung

Die der Erfindung zugrundeliegende Aufgabe wird nun durch eine pharmazeutische Zubereitung gelöst, die aus Cyclosporin A und D-α-Tocopherolpolyethylenglykol-1000-Succinat und zusätzlich einem Emulgator besteht oder sie enthält. Ein Gehalt an Pflanzenöl oder Fett beispielsweise gemäß Inn. Med., 19(1992) 52-55 ist dabei ausgenommen.

Die pharmazeutische Zubereitung kann ferner durch einen Gehalt an D-α-Tocopherolpolyethylenglykol-1000-Succinat von bis zu 900 % auf Basis von Cyclosporin A gekennzeichnet sein.

Die pharmazeutische Zubereitung kann ferner durch einen Gehalt an Cyclosporin A ≥ 10 % auf Basis der Zusammensetzung gekennzeichnet sein.

Die pharmazeutische Zubereitung kann ferner durch seinen Gehalt an Ethanol oder Isopropanol als Verarbeitungshilfsmittel insbesondere in Mengen bis 20 % gekennzeichnet sein.

Die pharmazeutische Zubereitung kann ferner durch einen Gehalt an Verdickungsmittel gekennzeichnet sein.

Die pharmazeutische Zubereitung kann schließlich in Form eines Injektionskonzentrats für Parenteralia mit dafür üblichen physiologisch verträglichen Hilfsstoffen vorgesehen werden.

Erfindungsgemäß wurde also überraschend gefunden, daß D-α-Trocopherolpolyethylenglycol-1000-Succinat ein ausgezeichnetes Lösungsvermögen für Cyclosporin A aufweist, gleichzeitig die Synthese von Prostanoiden wie Prostaglandinen und Thromboxanen hemmt, was zur Reduktion der Nephrotoxizität und zum Abklingen von Entzündungsreaktionen in der Haut ausgenützt werden kann und gleichzeitig die Resorption von Cyclosporin A durch die intakte Haut fördert. Weiterhin können erfindungsgemäße Lösungen, gegebenenfalls unter Zuhilfenahme von physiologisch verträglichen üblichen Hilfsstoffen zur Herstellung von Parenteralia zu Injektionskonzentraten formuliert werden. Der besondere Vorteil der erfindungsgemäßen Lösungen besteht neben der Erzielung hoher Konzentrationen an gelöstem Cyclosporin A von über 10 % darin, daß D-α-Tocopherolpolyethylenglykol-1000-Succinat Eigenwirkungen besitzt, die einerseits toxischen Wirkungen von Cyclosporin A bei den üblichen hohen Dosen bei oraler und parenteraler Anwendung entgegenwirken und andererseits den beabsichtigten immunsuppressiven Effekt bei der topischen Behandlung der Psoriasis über die resorptionsfördernde Wirkung verstärken.

Cyclosporin A lost sich nun völlig unerwartet in einer so hohen Konzentration > 10% in erfindungsgemäßen Zubereitungen mit einem Gehalt an D-α-Tocopherolpolyethylenglykol-1000-Succinat, so daß die Kombination als Lösung therapeutisch sinnvoll sowohl in Weichgelatinekapseln als auch in topischen Formulierungen einsetzbar ist. Die Formulierungen können dabei zur Unterstützung der Verarbeitbarkeit geringe Mengen Alkohol wie Ethanol oder Isopropanol in Mengen bis 20% der Gesamtformulierung enthalten.

Zur Förderung der Benetzung enthalten die erfindunggemäßen Zubereitungen physiologisch verträgliche oberflächenaktive Stoffe, das heißt Emulgatoren. Sowohl oral als topisch verträgliche Emulgatoren sind beispielsweise Phospholipide wie Lecithine, Lysolecithine, Ethoxylierungsproduckte von Fettsäuren und Fetten, Alkaliseifen, Sucroseester und andere, ohne darauf beschränkt zu sein.

Des weiteren können die Formulierungen Verdickungsmittel wie kolloidale Kieselsäure oder Polyacrylsäure oder Polyacrylsäurederivate oder Cellulosederivate sowie Antioxidantien und Geschmackshilfsstoffe enthalten.

### 5. Beispiele

| **Beispiel 1:** Zusammensetzung für eine Weichgelatinekapsel | |
|---|---|
| Cyclosporin A | 125 mg |
| Ethanol abs. | 125 mg |
| D-α-Tocopherol | 325 mg |
| D-α-Tocopherylethylenglycol-1000-Succinat | 425 mg |

## Patentansprüche

1. Pharmazeutische Zubereitung, bestehend aus oder enthaltend Cyclosporin A, Tocopherolpolyethylenglykol-1000-Succinat und zusätzlich einen Emulgator, wobei ein Gehalt an Öl oder Fett ausgenommen ist.

2. Pharmazeutische Zubereitung nach Anspruch 1, *gekennzeichnet* durch einen Gehalt Tocopherolpolyethylenglykol-1000-Succinat oder an einem seiner Derivate von bis zu 900 % auf Basis von Cyclosporin A.

3. Pharmazeutische Zubereitung nach einem der vorhergehenden Ansprüche, *gekennzeichnet* durch einen Gehalt an Cyclosporin A ≥ 10 % auf Basis der Zusammensetzung.

4. Pharmazeutische Zubereitung nach einem der vorhergehenden Ansprüche, *gekennzeichnet* durch seinen Gehalt an Ethanol oder Isopropanol als Verarbeitungshilfsmittel insbesondere in Mengen bis 20 %.

5. Pharmazeutische Zubereitung nach einem der vorhergehenden Ansprüche, *gekennzeichnet* durch einen Gehalt an Verdickungsmittel.

6. Pharmazeutische Zubereitung nach einem der vorhergehenden Ansprüche in Form eines Injektionskonzentrats für Parenteralia mit dafür üblichen physiologisch verträglichen Hilfsstoffen.

## Claims

1. Pharmaceutical preparation, consisting of or containing cyclosporin A, tocopherol polyethylene glycol 1000 succinate and in addition an emulgator, a content of oil or fat being excluded.

2. Pharmaceutical preparation according to claim 1, characterized by a content of tocopherol polyethylene glycol 1000 succinate or one of its derivatives of up to 900% on the basis of cyclosporin A.

3. Pharmaceutical preparation according to one of the preceding claims, characterized by a content of cyclosporin A of ≥ 10% on the basis of the composition.

4. Pharmaceutical preparation according to one of the preceding claims, characterized by its content of ethanol or isopropanol as processing auxiliaries, in particular in amounts of up to 20%.

5. Pharmaceutical preparation according to one of the preceding claims, characterized by a content of thickener.

6. Pharmaceutical preparation according to one of the preceding claims in the form of an injection concentrate for preparations for parenteral use with physiologically tolerable auxiliaries customary therefor.

## Revendications

1. Composition pharmaceutique constituée par ou comprenant une cyclosporine A, un succinate de tocophérol polyéthyléne glycol 1000 ainsi qu'un émulsifiant, une teneur en huile ou graisse étant exclue.

2. Composition pharmaceutique selon la revendication 1, caractérisée par une teneur en succinate de tocophérol polyéthyléne glycol 1000 ou de l'un de ses dérivés allant jusqu'à 900 % sur la base de cyclosporine A.

3. Composition pharmaceutique selon l'une des revendications précédentes, caractérisée par une teneur en cyclosporine A supérieure ou égale à 10 % sur la base de la composition.

4. Composition pharmaceutique selon l'une des revendications précédentes, caractérisée par sa teneur en éthanol ou isopropanol comme produit auxiliaire de fabrication, en particulier dans des quantités allant jusqu'à 20 %.

5. Composition pharmaceutique selon l'une des revendications précédentes, caractérisée par la présence d'un épaississant.

6. Composition pharmaceutique selon l'une des revendications précédentes sous forme d'un concentré injectable avec des adjuvants usuels physiologiquement tolérables pour une utilisation parentérale.
